(19) Europäisches Patentamt
European Patent Office
Office européen des brevets

(11) Veröffentlichungsnummer: **0 169 495**
**A2**

(12) # EUROPÄISCHE PATENTANMELDUNG

(21) Anmeldenummer: **85108934.2**

(22) Anmeldetag: **17.07.85**

(51) Int. Cl.[4]: **C 07 D 285/30**
A 61 K 31/54

(30) Priorität: **25.07.84 DE 3427309**

(43) Veröffentlichungstag der Anmeldung:
**29.01.86 Patentblatt 86/5**

(84) Benannte Vertragsstaaten:
**AT BE CH DE FR GB IT LI LU NL SE**

(71) Anmelder: **BOEHRINGER INGELHEIM KG**

**D-6507 Ingelheim am Rhein(DE)**

(84) Benannte Vertragsstaaten:
**BE CH DE FR IT LI LU NL SE AT**

(71) Anmelder: **Boehringer Ingelheim International G.m.b.H**

**D-6507 Ingelheim am Rhein(DE)**

(84) Benannte Vertragsstaaten:
**GB**

(72) Erfinder: **Köppe, Herbert, Dr.**
**Neuweg 72**
**D-6507 Ingelheim/Rhein(DE)**

(72) Erfinder: **Abele, Wolfgang, Dr.**
**Kirchstrasse 2a**
**D-6531 Waldalgesheim(DE)**

(72) Erfinder: **Esser, Franz, Dr.**
**Posener Strasse 30**
**D-6507 Ingelheim/Rhein(DE)**

(72) Erfinder: **Gaida, Wolfram, Dr.**
**Paul-Clemen-Strasse 14**
**D-6507 Ingelheim/Rhein(DE)**

(72) Erfinder: **Hoefke, Wolfgang, Dr.**
**Rosselstrasse 21**
**D-6200 Wiesbaden(DE)**

(54) **Neue 1-Aryloxypropanolamine, Verfahren zu ihrer Herstellung und ihre Verwendung.**

(57) Die neuen Verbindungen der Formel

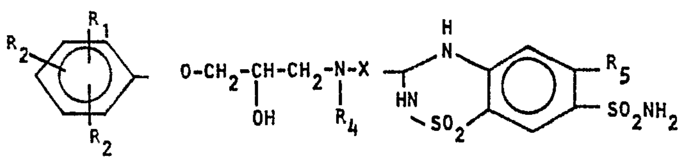

(I),

(die Definitionen der einzelnen Reste sind in der Beschreibung aufgeführt) können nach konventionellen Methoden hergestellt werden. Die Verbindungen eignen sich zur Behandlung und Prophylaxe des Bluthochdrucks.

EP 0 169 495 A2

Die Erfindung betrifft neue 1-Aryloxypropanolamine, ihre Herstellung nach an sich bekannten Verfahren und die Verwendung der neuen Verbindungen in der Therapie, insbesondere zur Behandlung oder Prophylaxe des Bluthochdrucks.

Die neuen 1-Aryloxypropanolamine entsprechen der allgemeinen Formel

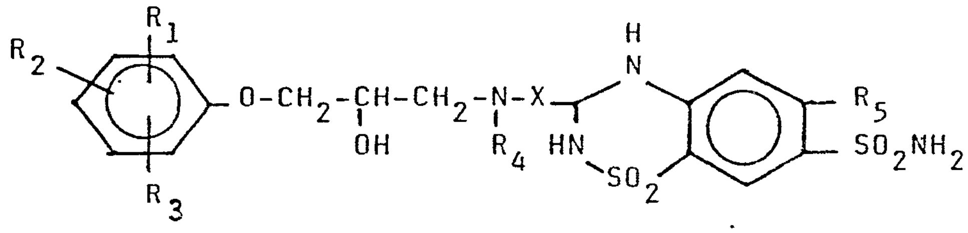

(I),

in der

$R_1$ ein Wasserstoff- oder Halogenatom, eine Niederalkyl-, Niederalkoxy-, Niederalkoxiniederalkyl-, Niederalkenyl-, Niederalkenyloxi-, Cyano-, Hydroxi-, Trifluormethyl-, Nitro-, Amino-, Aminocarbonylmethyl-, Mononiederalkylamino-, Diniederalkylaminogruppe,

$R_2$ ein Wasserstoff- oder Halogenatom, eine Cyano-, Niederalkyl- oder Niederalkoxigruppe oder eine Acylaminogruppe,

$R_3$ ein Wasserstoff- oder Halogenatom, eine Niederalkyl- oder Niederalkoxigruppe
$R_2$ und $R_3$ gemeinsam auch eine Butadienyl- oder eine Methylendioxigruppe,

$R_4$ ein Wasserstoffatom oder eine Niederalkylgruppe,

$R_5$ ein Chloratom oder eine Trifluormethylgruppe und

X eine Alkylengruppe

bedeutet, und ihre Säureadditionssalze.

Unter "Halogenatome" werden hier Fluor, Chlor und Brom verstanden. Bevorzugt sind Fluor und vor allem Chlor.

Die Niederalkyl- bzw. Niederalkoxigruppen enthalten 1 bis 4, vorzugsweise 1 bis 3 C-Atome; hervorzuheben sind die Gruppen mit 1 bis 2 C-Atomen. Die Alkylengruppe X enthält 1 bis 8 C-Atome; bevorzugt weist X 1 bis 5 C-Atome, vor allem 1 bis 3 C-Atome auf. Soweit die C-Ketten aus mehreren C-Atomen bestehen, können sie unverzweigt oder verzweigt sein. Dies gilt auch für den Acylrest in der Acylaminogruppe (Definition von $R_2$). Die Acylgruppe enthält dabei 1 bis 8, vorzugsweise 1 bis 4 C-Atome und kann aliphatisch oder aromatisch sein. Unter einem "aromatischen Rest" wird hier ein Phenyl-, Mono- oder Dimethylphenylrest, aber auch ein Benzylrest verstanden.

Enthält die Verbindung der Formel I mehrere Substituenten mit Kohlenstoffketten, so kann die Zahl der Kohlenstoffatome in diesen Ketten gleich oder verschieden sein.

Die Verbindungen der Formel I können als freie Basen oder als Säureadditionssalze vorliegen. Aufgrund ihrer Asymmetriezentren können sie jeweils in Form von Racematen bzw. von Gemischen von Anantiomeren oder in Form der einzelnen Enantiomeren vorliegen.

Geeignete Verfahren zur Herstellung der neuen Verbindungen werden im folgenden aufgeführt.

a) Umsetzung einer Verbindung der Formel

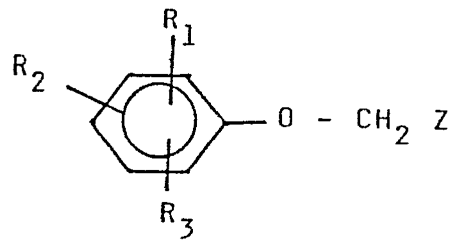

(II),

in der $R_1$, $R_2$ und $R_3$ die obige Bedeutung haben und Z die Gruppe 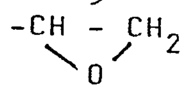 oder 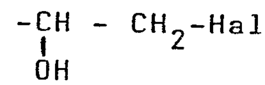

(Hal = Halogen, vorzugsweise Cl, Br)
bedeutet,

mit einer Verbindung der Formel

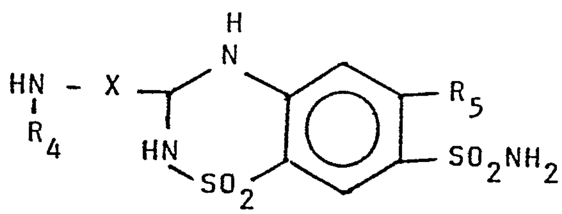

(III),

in der $R_4$, $R_5$ und X die obige Bedeutung haben.

b) Umsetzung einer Verbindung der Formel

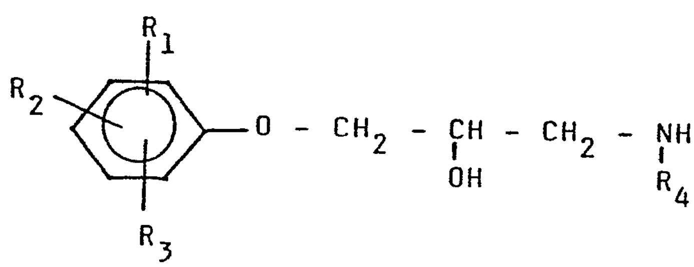

(IV)

in der $R_1$ bis $R_4$ die obige Bedeutung haben, mit einer Verbindung der Formel

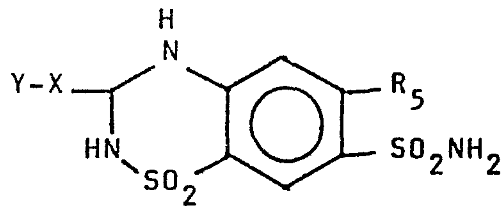

(V),

in der $R_5$ und X die obige Bedeutung haben und Y einen unter Verknüpfung von IV und V abspaltbaren Säurerest, (z.B. Halogenatom, insbesondere Cl, Br, oder Sulfonsäurerest) darstellt.

c) Zur Herstellung von Verbindungen der Formel I, in denen $R_4$ einen Niederalkylrest bedeutet:

Umsetzung solcher Verbindungen der Formel I, in denen $R_4$ Wasserstoff bedeutet, mit einer zur Einführung von $R_4$ gleich Niederalkyl in die Aminogruppe geeigneten Verbindung

$$Y - R_4 \qquad \text{(VI)},$$

in der $R_4$ und Y die obige Bedeutung haben.

Soweit nach den Verfahren a) bis c) zunächst Säureadditionssalze erhalten werden, werden diese nach üblichen Methoden gewünschtenfalls in freie Basen oder in Salze anderer Säuren übergeführt; zunächst erhaltene Basen werden gewünschtenfalls in Säureadditionssalze umgewandelt.

Verfahren a) wird vorzugsweise bei Temperaturen zwischen 0 und 40°C, insbesondere bei Raumtemperatur durchgeführt. Als Reaktionsmedium dienen Alkohole oder andere polare Lösungsmittel, z.B. Methanol, Ethanol, Isopropanol, Tetrahydrofuran, Dioxan, gegebenenfalls in Mischung.

Als Säurefänger können tertiäre Amine (z.B. Triethylamin, Dimethylanilin, Pyridin) oder anorganische Salze (z.B. $NaHCO_3$, $Na_2CO_3$, $K_2CO_3$) oder wäßrige Basen (z.B. Natronlauge, Kalilauge) verwendet werden.

Verfahren b) und c) wird vorzugsweise bei Temperaturen zwischen Raumtemperatur und der Siedetemperatur des Reaktionsgemischs, insbesondere bei Temperaturen zwischen 40 und 80°C durchgeführt. Als Reaktionsmedium können polare Lösungsmittel dienen, z.B. Dimethylformamid, Tetrahydrofuran, Dioxan, Methanol, Ethanol, Propanole. Als Säurefänger dienen Salze wie $NaHCO_3$, $KHCO_3$, $Na_2CO_3$ oder tertiäre Amine.

Die Ausgangsverbindungen der allgemeinen Formeln II bis VI können nach bekannten Methoden hergestellt werden.

Die erfindungsgemäßen Verbindungen besitzen zwei asymmetrische C-Atome und kommen daher in den verschiedenen diastereoisomeren Formen vor.

Die erfindungsgemäßen 1-Aryloxi-3-dihydrobenzothiadiazinylalkylamino-2-propanole der allgemeinen Formel I können in üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure oder Weinsäure.

Die Verbindungen der allgemeinen Formel I bzw. deren physiologisch verträgliche Säureadditionssalze haben im Tierversuch wertvolle therapeutische, insbesondere blutdrucksenkende Eigenschaften gezeigt und können daher beispielsweise zur Behandlung oder Prophylaxe des Bluthochdrucks in der Humanmedizin eingesetzt werden.

Als wertvoll haben sich dabei insbesondere solche Verbindungen der allgemeinen Formel I herausgestellt, bei denen $R_1$ und $R_2$ jeweils ein Chloratom oder $R_1$ einen Cyanorest in 2-Position und $R_2$ einen Acylaminorest in 4-Position oder $R_1$ einen Alkoxialkylrest in 4-Position bedeutet.

Die Einzeldosis der erfindungsgemäßen Substanzen liegt bei 5 bis 300 mg, vorzugsweise bei 20 bis 150 mg (oral).

Die erfindungsgemäßen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyçlamat, Glycerin oder Zucker, sowie ein geschmackverbesserndes Mittel, z.B. Aromastoffe, wie Vanillin oder Organgenextrakt, enthalten.

Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Koservierungsmitteln, wie p-Hydroxibenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemäßen Verbindungen sind auch für die Kombination mit anderen pharmakodynamisch wirksamen Stoffen wie z.B. ß-Adrenolytika, Diuretika oder Tranquilizern geeignet.

Die folgenden Beispiele erläutern die Erfindung, ohne sie zu beschränken:

## Herstellungsbeispiele

### Beispiel 1

**1-(2,4-Dichlorphenoxi)-3-[(6-Chlor-7-sulfonamido-1,1-dioxo-dihydrobenzothiadiazinyl-3)-methylamino]-2-propanol-hydrochlorid**

7,3 g (0,02 Mol) 3-Aminomethyl-6-chlor-7-sulfonamido-1,1-dioxo-dihydro-benzothiadiazin-hydrochlorid werden in einer Mischung aus 70 ml Äthanol und 30 ml Methanol gelöst, 2 g (0,02 Mol) Triäthylamin zugegeben und unter Rühren 4,4 g (0,02 Mol) 1-(2,4-Dichlorphenoxi)-2,3-propylenoxid zugegeben. Nach 48-stündigem Stehen bei 20$^{o}$ wurde das Lösungsmittelgemisch unter verminderten Druck abdestilliert, der verbleibende Rückstand über eine Kieselgelsäule gereinigt. Laufmittel: 700 Teile Essigester, 300 Teile Isopropanol, 50 Teile Ammoniak. Nach Vereinigung der einheitlichen Fraktionen und Abdestillieren des Lösungsmittelgemisches verbleiben 7,1 g Reinsubstanz, die in 50 ml Acetonitril gelöst wurden. Nach Zugabe von alkoholischer HCl kristallisierte das Hydrochlorid in farbloser Form aus. Nach Isolierung und Trocknung wurden 2,8 g farbloses Kristallisat gewonnen. Fp: 170 - 173$^{o}$. Aus der Mutterlauge kristallisierten nach Einengen nochmals 0,7 g Substanz aus.

### Beispiel 2

**1-(2-Cyanophenoxi)-3-[(6-chlor-7-sulfonamido-1,1-dioxo-dihydrobenzothiadiazinyl-3)-methylamino]-2-propanol-hydrochlorid**

9 g (0,03 Mol) 3-Aminomethyl-6-chlor-7-sulfonamido-1,1-dioxo-dihydro-benzothiadiazin-hydrochlorid werden nach Lösen in 80 ml Äthanol und Zugabe von 20 ml Methanol mit 2,53 g (0,02 Mol) Triäthylamin vereinigt. Bei Raumtemperatur werden 4,35 g (0,02 Mol) 1-(2-Cyanophenoxi)-2,3-propanepoxid zugegeben und zwölf

Stunden stehen gelassen. Nach Abdestillieren der Lösungsmittel im Vakuum wird der Rückstand über eine Kieselgelsäule gereinigt. Lösungsmittelgemisch: 700 Teile Essigester, 300 Teile Isopropanol, 50 Teile Ammoniak. Die vereinigten einheitlichen Fraktionen ergaben nach Abdestillieren der flüchtigen Anteile 3,9 g Base, die in Äthanol gelöst und mit ätherischer HCl angesäuert wurde. Nach Filtration wurde Äther bis zur leichten Trübung zugegeben, wonach sich die Substanz farblos abschied. Nach Absaugen erhielt man 2,1 g Hydrochlorid vom Schmelzpunkt. Fp: 170 - 172$^o$.

In Analogie zu Beispiel 2 wurden die Verbindungen der Beispiele 3 bis 14 synthetisiert:

Beispiel 3

1-[4-(2-Methoxiäthyl)-phenoxi)-3-[(6-chlor-7-sulfonamido-1,1-dioxo-dihydrobenzothiadiazinyl-3)-methylamino]-2-propanol-hydrochlorid

Fp: 172 - 174$^o$.

Beispiel 4

1-α-Naphthoxi-3-[(6-chlor-7-sulfonamido-1,1-dioxo-dihydrobenzothiadiazinyl-3)-methylamino]-2-propanol-hydrochlorid

Fp: 196 - 199$^o$.

Beispiel 5

1-(3-Methylphenoxi)-3-[(6-chlor-7-sulfonamido-1,1-dioxo-dihydrobenzothiadiazinyl-3)-methylamino]-2-propanol-hydrochlorid

Fp: 188 - 190$^o$.

Beispiel 6

1-(2-Allyloxiphenoxi)-3-[(6-chlor-7-sulfonamido-1,1-dioxo-dihydrobenzothiadiazinyl-3)-methylamino]-2-propanol-hydrochlorid

Fp: 120 - 122°.

Beispiel 7

1-(2-Cyano-4-isobutyroylaminophenoxi)-3-[(6-chlor-7-sulfonamido-1,1-dioxi-dihydrobenzothiadiazinyl-3)-methylamino]-2-propanol-hydrochlorid

Fp: 190 - 193°.

Beispiel 8

1-(4-Chlorphenoxi)-3-[(6-chlor-7-sulfonamido-1,1-dioxo-dihydrobenzothiadiazinyl-3)-methylamino]-2-propanol-hydrochlorid

Fp: 186 - 187°.

Beipiel 9

1-(2,6-Dichlorphenoxi)-3-[(6-chlor-7-sulfonamido-1,1-dioxo-dihydrobenzothiadiazinyl-3)-methylamino]-2-propanol-hydrochlorid

Fp: 184 - 186°.

Beispiel 10

1-(3,4-Dichlorphenoxi)-3-[(6-chlor-7-sulfonamido-1,1-dioxo-dihydrobenzothiadiazinyl-3)-methylamino]-2-propanol-oxalat

Fp: 140 - 142°.

Beispiel 11

1-(3-Methoxiphenoxi)-3-[(6-chlor-7-sulfonamido-1,1-dioxo-dihydrobenzothiadiazinyl-3)-methylamino]-2-propanol-hydrochlorid

Fp: 184 - 186°.

Beispiel 12

1-(4-Methoxiphenoxi)-3-[(6-chlor-7-sulfonamido-1,1-dioxo-dihydrobenzothiadiazinyl-3)-methylamino]-2-propanol-hydrochlorid

Fp: 188 - 189°.

Beispiel 13

1-(3-Trifluormethylphenoxi)-3-[(6-chlor-7-sulfonamido-1,1-dioxo-dihydrobenzothiadiazinyl-3)-methylamino]-2-propanol-hydrochlorid

Fp: 131 - 134°.

Beipiel 14

1-(3,4-Dimethoxiphenoxi)-3-[(6-chlor-7-sulfonamido-1,1-dioxo-dihydrobenzothiadiazinyl-3)-methylamino]-2-propanol-hydrochlorid

Fp: 178 - 180°.

Beipiel 15

1-(2-Cyano-4-isobutyroylamino-phenoxi)-3-N-methyl-3-[(6-chlor-7-sulfonamido-1,1-dioxo-dihydrobenzothiadiazinyl-3)-methylamino]-2-propanol-oxalat

7,5 g (0,2 Mol) 3-N-Methylaminomethyl-6-chlor-7-sulfonamido-1,1-dioxo-dihydro-benzothiadiazin-hydrochlorid werden in

100 ml Methanol gelöst und nach Zugabe von 2,75 ml (0,02 Mol) Triäthylamin mit 5,2 g (0,02 Mol) 1-(2-Cyano-4-isobutyroylamino-phenoxi)-2,3-propanepoxid bei Raumtemperatur umgesetzt. Nach zwölfstündigem Stehen wird das Lösungsmittel unter Vakuum abdestilliert und der verbleibende Rückstand über eine Kieselgelsäule wie in Beispiel 2 gereinigt. Die so erhaltene Base wird in Aceton gelöst und in eine Lösung von 3,5 g Oxalsäure in 150 ml Aceton unter Rühren langsam eingetropft. Das Oxalat kristalliert nach Zugabe von Äther langsam aus. Nach Isolierung und Trocknung werden 2,8 g farblose Reinsubstanz erhalten. Fp: 138 - 142$^{o}$.

Analog zu Beispiel 15 wurden die Verbindungen der Beispiele 16 bis 21 hergestellt.

Beispiel 16

1-(2-Cyanophenoxi)-3-N-methyl-3-[(6-chlor-7-sulfonamido-1,1-dioxo-dihydrobenzothiadiazinyl-3)-methylamino]-2-propanol-oxalat

Fp: 127 - 130$^{o}$.

Beispiel 17

1-(3,4-Dimethoxiphenoxi)-3-N-methyl-3-[(6-chlor-7-sulfonamido-1,1-dioxo-dihydrobenzothiadiazinyl-3)-methylamino]-2-propanol-oxalat

Fp: 133 - 136$^{o}$.

Beispiel 18

1-(2,4-Dichlorphenoxi)-3-N-methyl-3-[(6-chlor-7-sulfonamido-1,1-dioxo-dihydrobenzothiadiazinyl-3)-methylamino]-2-propanol-hydrochlorid

Fp: 174 - 177$^{o}$.

Beispiel 19

1-(3-Methylphenoxi)-3-N-methyl-3-[(6-chlor-7-sulfonamido-1,1-dioxo-dihydrobenzothiadiazinyl-3)-methylamino]-2-propanol-oxalat

Fp: 143 - 146°.

Beispiel 20

1-(2,4-Dichlorphenoxi)-3-N-isopropyl-3-[(6-chlor-7-sulfonamido-1,1-dioxo-dihydrobenzothiadiazinyl-3)-methylamino]-2-propanol-hydrochlorid

Fp: 175 - 177°.

Beispiel 21

1-(2-Cyanophenoxi)-3-N-isopropyl-3-[(6-chlor-7-sulfonamido-1,1-dioxo-dihydrobenzothiadiazinyl-3)-methylamino]-2-propanol-hydrochlorid

Fp: 179 - 181°.

Beispiel 22

1-(2-Cyanophenoxi)-3-[(6-chlor-7-sulfonamido-1,1-dioxo-dihydrobenzothiadiazinyl-3)-äthylamino]-2-propanol-hydrochlorid

7,54 g (0,02 Mol) 3-Aminoäthyl-6-chlor-7-sulfonamido-1,1-dioxo-dihydrobenzothiadiazin-hydrochlorid werden in 100 ml Methanol gelöst, 2,0 g (0,02 Mol) Triäthylamin zugegeben und 3,5 g (0,02 Mol) 1-(2-Cyanophenoxy)-2,3-propylenoxid eingerührt. Nach zwölfstündigem Stehen bei 20° werden die flüchtigen Teile im Vakuum abdestilliert. Der Rückstand wird

wie in Beispiel 2 über eine Kieselgelsäule gereinigt. Die isolierten basischen Anteile werden in Aceton gelöst. Die Lösung wird filtriert, mit alkoholischer Salzsäure angesäuert und zum Auskristallisieren 20 Stunden unter Kühlung stehen gelassen. Das farblose Hydrochlorid wird abgesaugt und getrocknet. Es werden 1,2 g Reinsubstanz gewonnen. Fp: 202 - 204°.

Entsprechend Beispiel 22 wurden noch folgende Verbindungen hergestellt:

Beispiel 23

1-(2,4-Dichlorphenoxi)-3-[(6-chlor-7-sulfonamido-1,1-dioxo-dihydrobenzothiadiazinyl-3)-äthylamino]-2-propanol-hydrochlorid

Fp: 187 - 190°.

Beispiel 24

1-(4-Methoxiäthylphenoxi)-3-[(6-chlor-7-sulfonamido-1,1-dioxo-dihydrobenzothiadiazinyl-3)-äthylamino]-2-propanol-oxalat

Fp: 137 - 140°.

Beipiel 25

1-(3,4-Dichlorphenoxi)-3-[(6-chlor-7-sulfonamido-1,1-dioxo-dihydrobenzothiadiazinyl-3)-äthylamino]-2-propanol-hydrochlorid

Fp: 188 - 190°.

Patentansprüche

1. Verbindungen der Formel

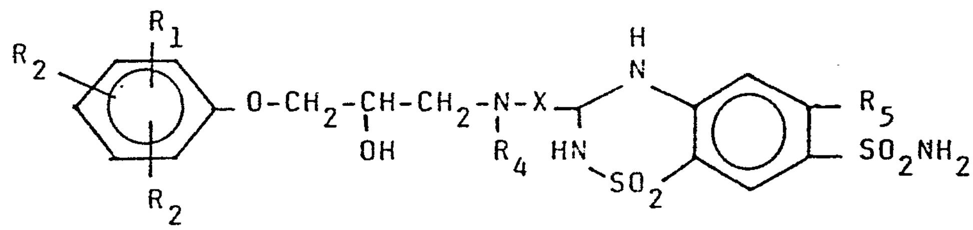

(I),

in der

$R_1$ ein Wasserstoff- oder Halogenatom, eine Niederalkyl-, Niederalkoxy-, Niederalkoxiniederalkyl-, Niederalkenyl-, Niederalkenyloxi-, Cyano-, Hydroxi-, Trifluormethyl-, Nitro-, Amino-, Aminocarbonylmethyl-, Mononiederalkylamino-, Diniederalkylaminogruppe,

$R_2$ ein Wasserstoff- oder Halogenatom, eine Cyano-, Niederalkyl- oder Niederalkoxigruppe oder eine Acylaminogruppe,

$R_3$ ein Wasserstoff- oder Halogenatom, eine Niederalkyl- oder Niederalkoxigruppe

$R_2$ und $R_3$ gemeinsam auch eine Butadienyl- oder eine Methylendioxigruppe,

$R_4$ ein Wasserstoffatom oder eine Niederalkylgruppe,

$R_5$ ein Chloratom oder eine Trifluormethylgruppe und

X eine Alkylengruppe

bedeutet, und ihre Säureadditionssalze.

2. Verbindungen nach Anspruch 1, in denen $R_1$ und $R_2$ für Chloratome stehen.

3. Verbindungen nach Anspruch 1, in denen $R_1$ für CN in 2-Position und $R_2$ für Acylamino in 4-Position steht.

4. Verbindungen nach Anspruch 1, in denen $R_1$ für eine Alkoxigruppe in 4-Position steht.

5. Arzneimittel, gekennzeichnet durch einen Gehalt an mindestens einer Verbindung nach Anspruch 1 bis 4.

6. Verwendung von Verbindungen nach Anspruch 1 bis 4 bei der Behandlung oder Prophylaxe des Bluthochdrucks.

7. Verfahren zur Herstellung von Verbindungen nach Anspruch 1 bis 4, dadurch gekennzeichnet, daß man

a) eine Verbindung der Formel

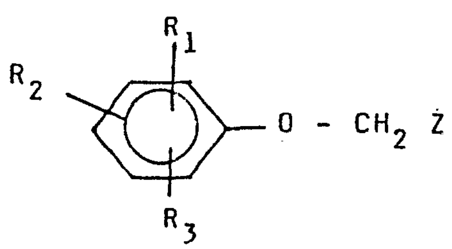

(II),

in der $R_1$, $R_2$ und $R_3$ die obige Bedeutung haben und Z die Gruppe 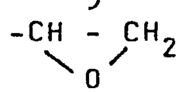 oder 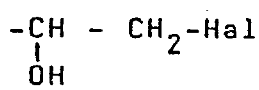

(Hal = Halogen, vorzugsweise Cl, Br) bedeutet,

mit einer Verbindung der Formel

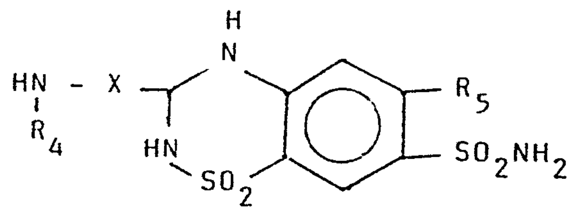

(III),

in der $R_4$ und $R_5$ die obige Bedeutung haben, umsetzt oder daß man

b) eine Verbindung der Formel

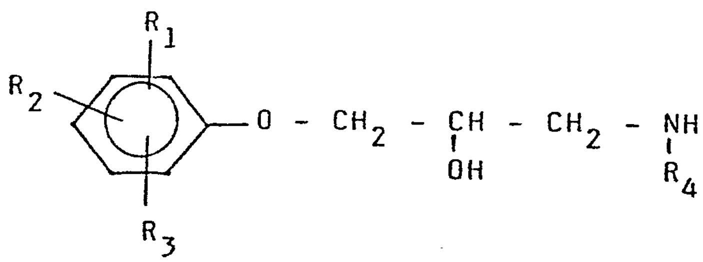

(IV)

in der $R_1$ bis $R_4$ die obige Bedeutung haben, mit einer Verbindung der Formel

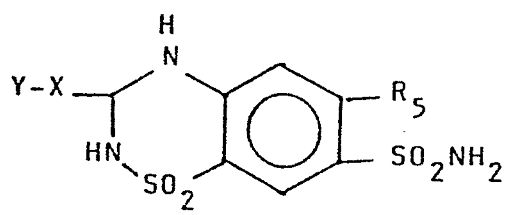

(V),

in der $R_5$ und X die obige Bedeutung haben und Y einen unter Verknüpfung von IV und V abspaltbaren Säurerest, (z.B. Halogenatom, insbesondere Cl, Br, oder Sulfonsäurerest) darstellt, umsetzt

oder daß man

c) zur Herstellung solcher Verbindungen der Formel I, in denen $R_4$ einen Niederalkylrest bedeutet, solche Verbindungen der Formel I, in denen $R_4$ Wasserstoff bedeutet, mit einer zur Einführung von $R_4$ gleich Niederalkyl in die Aminogruppe geeigneten Verbindung

$$Y - R_4 \qquad (VI),$$

in der $R_4$ und Y die obige Bedeutung haben, umsetzt

und daß man gewünschtenfalls nach den Verfahren a) bis c) erhaltene Säureadditionssalze in freie Basen oder in andere Säureadditionssalze überführt oder zunächst erhaltene freie Basen in Säureadditionssalze umwandelt.